# EUROPEAN PATENT SPECIFICATION

(11) **EP 3 766 079 B1**
(45) Date of publication and mention of the grant of the patent: **16.09.2026**
(21) Application number: 19710378.1
(22) Date of filing: 08.03.2019
(51) Int. Cl.: G16H 30/40, A61B 8/08, G06T 7/00

(54) **SCORING INTRAVASCULAR LESIONS AND STENT DEPLOYMENT IN MEDICAL INTRALUMINAL ULTRASOUND IMAGING**
BEWERTUNG VON INTRAVASKULÄREN LÄSIONEN UND STENTEINFÜHRUNG IN DER MEDIZINISCHEN INTRALUMINALEN ULTRASCHALLBILDGEBUNG
NOTATION DE LÉSIONS INTRAVASCULAIRES ET DÉPLOIEMENT D'ENDOPROTHÈSE DANS UNE IMAGERIE ULTRASONORE INTRALUMINALE MÉDICALE

(30) Priority: 14.03.2018 US 201862643105 P
(43) Date of publication of application: 20.01.2021
(73) Proprietor: Koninklijke Philips N.V., 5656 AE Eindhoven (NL)
(72) Inventor: MERRITT, Fergus, 5656 AE Eindhoven (NL); NAIR, Anuja, 5656 AE Eindhoven (NL)
(74) Representative: Philips Intellectual Property & Standards
(86) International application number: PCT/EP2019/055795
(87) International publication number: WO 2019/175032

(56) References cited:
- EP-A1- 3 466 326
- US-A1- 2008 103 389
- US-A1- 2014 257 087
- US-A1- 2016 157 787
- US-A1- 2016 157 802
- US-A1- 2016 174 925
- US-A1- 2016 292 857
- US-A1- 2019 102 906

## Description

### TECHNICAL FIELD

The present disclosure relates generally to medical intraluminal ultrasound imaging, including imaging associated with a body lumen of a patient using an intraluminal imaging device. For example, the present disclosure describes scoring the severity of lesions or the expansion of stents by analyzing intraluminal images (e.g., intravascular ultrasound or IVUS images).

### BACKGROUND

Various types of intraluminal (also referred to as intravascular) imaging systems are used in diagnosing and treating diseases. For example, intravascular ultrasound (IVUS) imaging is widely used in interventional cardiology as a diagnostic tool for visualizing vessels within a body of a patient. This may aid in assessing diseased vessels, such as an arteries, within the human body to determine the need for treatment, to optimize treatment, and/or to assess its effectiveness.

In some cases, intraluminal imaging is carried out with an IVUS device including one or more ultrasound transducers. The IVUS device may be passed into the vessel and guided to the area to be imaged. The transducers emit ultrasonic energy and receive ultrasound echoes reflected from the vessel. The ultrasound echoes are processed to create an image of the vessel of interest. The image of the vessel of interest may include one or more lesions or blockages in the vessel. A stent may be placed within the vessel to treat these blockages and intraluminal imaging may be carried out to view the placement of the stent within the vessel.

A medical professional may need to score the severity of lesions within the vessel based on the images of the vessel to determine if the lesions are critical, moderate, or insignificant. Typically, the medical professional must make this determination by visually comparing images of the vessel to identify a lesion and then deciding on the severity of the lesion. This may lead to logistical and judgment errors because the medical professional may miss a lesion completely or misjudge the severity of the lesion. The medical professional may also be required to determine the effectiveness of a stent with in a lumen by simply viewing images of the stent within the lumen. These current methods may be time consuming and may lead to errors in determining lesion severity and stent efficiency. Thus, deficiencies exist in current intraluminal image scoring systems.
US2014/257087 A1 discloses an optical coherence tomography (OCT) system that includes a processor, memory, a stent detection software component and a stent malapposition detection component. A given stent and components thereof can be visualized to include an indicia or measurement of the degree of contact or lack of contact between one or more stent struts, which are components of the stent, and the wall of a blood vessel. One area showing a degree of stent malapposition with respect to stent and wall is shown in a region.
US2016/292857 A1 discloses an OCT system comprising algorithms to detect stent location within the polar coordinate system using features within one-dimensional images, such as A-scan, two-dimensional images, such as a B-scan, and/or three-dimensional images. Once the polar coordinates of the object are detected, the polar coordinates are converted to the Cartesian coordinates and displayed as a tomographic image. Thus, a three-dimensional profile of the stent can be detected and displayed to a user. In addition, with the polar coordinates of the stent automatically detected, the stent strut apposition or coverage relative to the lumen border is computed.

### SUMMARY

Systems, devices, and methods for evaluating a blockage within a body lumen (e.g., a lesion within a blood vessel), as well as for evaluating a treatment for the blockage (e.g., a stent within the blood vessel) are disclosed. In particular, the intraluminal image scoring system may provide automated scoring of the severity of lesions and the expansion of stents. The lesion score or expansion score may be provided with one or more images of a vessel on a display device. The lesion score and expansion score may assist a user in quickly and accurately assessing a lumen and/or stent for further imaging procedures or treatment.

The invention is defined by the claims. Aspects of the present invention advantageously provide intraluminal scoring systems and analysis that overcome the limitations of existing intraluminal scoring systems.

An intraluminal imaging system according to claim 1 is provided.

### BRIEF DESCRIPTION OF THE DRAWINGS

Illustrative embodiments of the present disclosure will be described with reference to the accompanying drawings, of which:
Fig. 1 is a diagrammatic schematic view of an intraluminal imaging system, according to aspects of the present disclosure.
Fig. 2 is an exemplary illustration of a display showing a prompt according to aspects of the present disclosure.
Fig. 3 is an exemplary illustration of a display showing another prompt according to aspects of the present disclosure.
Fig. 4 is an exemplary illustration of a display showing another prompt and instructions according to aspects of the present disclosure.
Fig. 5 is an exemplary illustration of a display showing imaging data and instructions according to aspects of the present disclosure.
Fig. 6 is an exemplary illustration of a display showing imaging data according to aspects of the present disclosure.
Fig. 7 is an exemplary illustration of a display showing various views of imaging data according to aspects of the present disclosure.
Fig. 8 is an exemplary illustration of a display showing imaging data and measurements according to aspects of the present disclosure.
Fig. 9 is an exemplary illustration of another display showing imaging data and measurements according to aspects of the present disclosure.
Fig. 10 is an exemplary illustration of a display showing imaging data and a lesion score according to aspects of the present disclosure.
Fig. 11 is an exemplary illustration of a display showing a stent and an expansion score according to aspects of the present disclosure.
Fig. 12 is a flow diagram of a method of determining and displaying a lesion score according to aspects of the present disclosure.
Fig. 13 is a flow diagram of a method of determining and displaying an expansion score according to aspects of the present disclosure.

### DETAILED DESCRIPTION

For the purposes of promoting an understanding of the principles of the present disclosure, reference will now be made to the embodiments illustrated in the drawings, and specific language will be used to describe the same. It is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

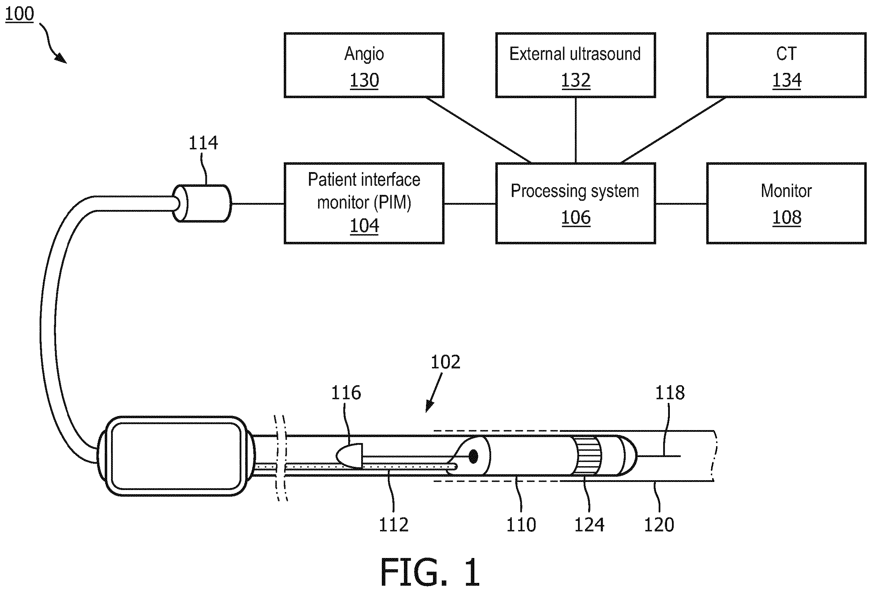

Fig. 1 is a diagrammatic schematic view of an intraluminal imaging system 100, according to aspects of the present disclosure. The intraluminal imaging system 100 can be an intravascular ultrasound (IVUS) imaging system in some embodiments. The intraluminal imaging system 100 may include an intraluminal device 102, a patient interface module (PIM) 104, a console or processing system 106, a monitor 108, an angiography system 130, an external ultrasound system 132, and/or a computed tomography (CT) system 134. The intraluminal device 102 is sized and shaped, and/or otherwise structurally arranged to be positioned within a body lumen of a patient. For example, the intraluminal device 102 can be a catheter, guide wire, guide catheter, pressure wire, and/or flow wire in various embodiments. In some circumstances, the system 100 may include additional elements and/or may be implemented without one or more of the elements illustrated in Fig. 1. For example, the system 100 may omit one or both of the external ultrasound system 132 and the CT system 134. In some embodiments, the intraluminal imaging system 100 is configured to provide scoring of a lesion and/or an expansion of a stent. These scoring methods may assist an operator in understanding the severity of a lesion or the effective expansion of a stent.

The intraluminal imaging system 100 (or intravascular imaging system) can be any type of imaging system suitable for use in the lumens or vasculature of a patient. In some embodiments, the intraluminal imaging system 100 is an intraluminal ultrasound (IVUS) imaging system. In other embodiments, the intraluminal imaging system 100 may include systems configured for forward looking intraluminal ultrasound (FL-IVUS) imaging, intraluminal photoacoustic (IVPA) imaging, intracardiac echocardiography (ICE), transesophageal echocardiography (TEE), and/or other suitable imaging modalities.

It is understood that the system 100 and/or device 102 can be configured to obtain any suitable intraluminal imaging data. In some embodiments, the device 102 can include an imaging component of any suitable imaging modality, such as optical imaging, optical coherence tomography (OCT), *etc.* In some embodiments, the device 102 can include any suitable imaging component, including a pressure sensor, a flow sensor, a temperature sensor, an optical fiber, a reflector, a mirror, a prism, an ablation element, a radio frequency (RF) electrode, a conductor, and/or combinations thereof. Generally, the device 102 can include an imaging element to obtain intraluminal data associated with the lumen 120. The device 102 may be sized and shaped (and/or configured) for insertion into a vessel or lumen 120 of the patient.

The system 100 may be deployed in a catheterization laboratory having a control room. The processing system 106 may be located in the control room. Optionally, the processing system 106 may be located elsewhere, such as in the catheterization laboratory itself. The catheterization laboratory may include a sterile field while its associated control room may or may not be sterile depending on the procedure to be performed and/or on the health care facility. The catheterization laboratory and control room may be used to perform any number of medical imaging procedures such as angiography, fluoroscopy, CT, IVUS, virtual histology (VH), forward looking IVUS (FL-IVUS), intraluminal photoacoustic (IVPA) imaging, a fractional flow reserve (FFR) determination, a coronary flow reserve (CFR) determination, optical coherence tomography (OCT), computed tomography, intracardiac echocardiography (ICE), forward-looking ICE (FLICE), intraluminal palpography, transesophageal ultrasound, fluoroscopy, and other medical imaging modalities, or combinations thereof. In some embodiments, device 102 may be controlled from a remote location such as the control room, such than an operator is not required to be in close proximity to the patient.

The intraluminal device 102, PIM 104, monitor 108, angiography system 130, external ultrasound system 132, and CT system 134 may be communicatively coupled directly or indirectly to the processing system 106. These elements may be communicatively coupled to the medical processing system 106 via a wired connection such as a standard copper link or a fiber optic link and/or via wireless connections using IEEE 802.11 Wi-Fi standards, Ultra Wide-Band (UWB) standards, wireless FireWire, wireless USB, or another high-speed wireless networking standard. The processing system 106 may be communicatively coupled to one or more data networks, e.g., a TCP/IP-based local area network (LAN). In other embodiments, different protocols may be utilized such as Synchronous Optical Networking (SONET). In some cases, the processing system 106 may be communicatively coupled to a wide area network (WAN). The processing system 106 may utilize network connectivity to access various resources. For example, the processing system 106 may communicate with a Digital Imaging and Communications in Medicine (DICOM) system, a Picture Archiving and Communication System (PACS), and/or a Hospital Information System via a network connection.

At a high level, the intraluminal device 102 emits ultrasonic energy from a transducer array 124 included in scanner assembly 110 mounted near a distal end of the intraluminal device 102. The ultrasonic energy is reflected by tissue structures in the medium (such as a lumen 120) surrounding the scanner assembly 110, and the ultrasound echo signals are received by the transducer array 124. The scanner assembly 110 generates electrical signal(s) representative of the ultrasound echoes. The scanner assembly 110 can include one or more single ultrasound transducers and/or a transducer array 124 in any suitable configuration, such as a planar array, a curved array, a circumferential array, an annular array, *etc.* For example, the scanner assembly 110 can be a one-dimensional array or a two-dimensional array in some instances. In some instances, the scanner assembly 110 can be a rotational ultrasound device. The active area of the scanner assembly 110 can include one or more transducer materials and/or one or more segments of ultrasound elements (*e.g*., one or more rows, one or more columns, and/or one or more orientations) that can be uniformly or independently controlled and activated. The active area of the scanner assembly 110 can be patterned or structured in various basic or complex geometries. The scanner assembly 110 can be disposed in a side-looking orientation (*e.g*., ultrasonic energy emitted perpendicular and/or orthogonal to the longitudinal axis of the intraluminal device 102) and/or a forward-looking looking orientation (*e.g*., ultrasonic energy emitted parallel to and/or along the longitudinal axis). In some instances, the scanner assembly 110 is structurally arranged to emit and/or receive ultrasonic energy at an oblique angle relative to the longitudinal axis, in a proximal or distal direction. In some embodiments, ultrasonic energy emission can be electronically steered by selective triggering of one or more transducer elements of the scanner assembly 110.

The ultrasound transducer(s) of the scanner assembly 110 can be a piezoelectric micromachined ultrasound transducer (PMUT), capacitive micromachined ultrasonic transducer (CMUT), single crystal, lead zirconate titanate (PZT), PZT composite, other suitable transducer type, and/or combinations thereof. In an embodiment the ultrasound transducer array 124 can include any suitable number of individual transducers between 1 transducer and 1000 transducers, including values such as 2 transducers, 4 transducers, 36 transducers, 64 transducers, 128 transducers, 500 transducers, 812 transducers, and/or other values both larger and smaller.

The PIM 104 transfers the received echo signals to the processing system 106 where the ultrasound image (including the flow information) is reconstructed and displayed on the monitor 108. The console or processing system 106 can include a processor and a memory. The processing system 106 may be operable to facilitate the features of the intraluminal imaging system 100 described herein. For example, the processor can execute computer readable instructions stored on the non-transitory tangible computer readable medium.

The PIM 104 facilitates communication of signals between the processing system 106 and the scanner assembly 110 included in the intraluminal device 102. This communication may include providing commands to integrated circuit controller chip(s) within the intraluminal device 102, select particular element(s) on the transducer array 124 to be used for transmit and receive, providing the transmit trigger signals to the integrated circuit controller chip(s) to activate the transmitter circuitry to generate an electrical pulse to excite the selected transducer array element(s), and/or accepting amplified echo signals received from the selected transducer array element(s) via amplifiers included on the integrated circuit controller chip(s). In some embodiments, the PIM 104 performs preliminary processing of the echo data prior to relaying the data to the processing system 106. In examples of such embodiments, the PIM 104 performs amplification, filtering, and/or aggregating of the data. In an embodiment, the PIM 104 also supplies high- and low-voltage DC power to support operation of the intraluminal device 102 including circuitry within the scanner assembly 110.

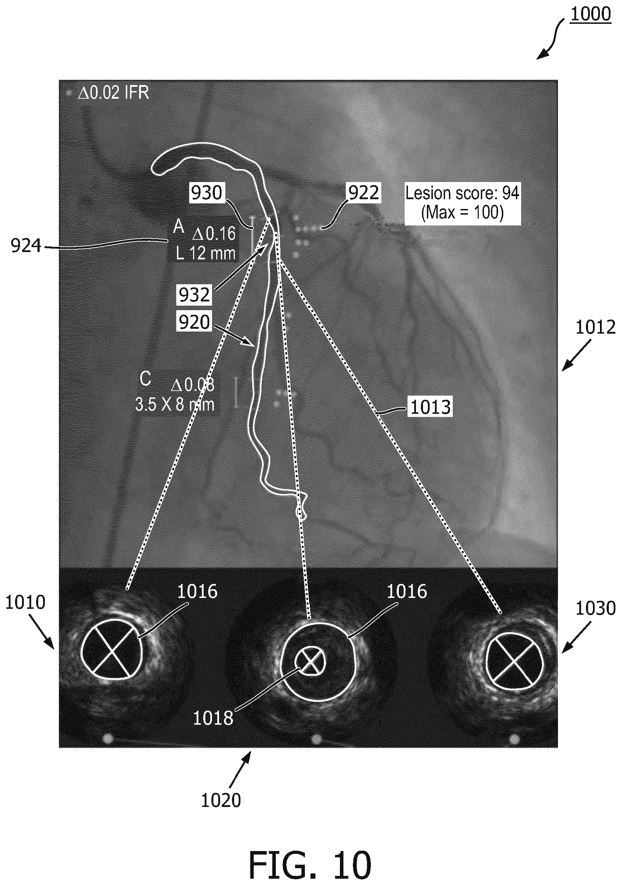

In some embodiments, the PIM 104 facilitates communications between the processing system 106 and one or more of the angiography system 130, the external ultrasound system 132, and/or the CT system 134. The angiography system 130 may include components configured to perform any of radiography, angiography, and fluoroscopy. In some circumstances, the angiography system 130 and/or the CT system 134 may be used to obtain images of the subject's vasculature which may be used as a reference for other imaging data. For example, an angiographic image may be displayed along with intraluminal imaging data (such as shown in Fig. 10) to provide better context for the images.

In some embodiments, the IVUS data and/or the external ultrasound data may be co-registered with the 2D or 3D CT image, which may further improve placement accuracy and decrease procedural time. The placement of the intraluminal device 102 may be verified with this multi-imaging system, which may improve outcomes versus standard fluoroscopic guidance. In some embodiments, the intraluminal device 102 is tracked to the target location as identified on a CT image and/or angiogram (such as a lesion or aneurysm). In some embodiments, a roadmap produced from co-registered IVUS and CT image data may be correlated to fluoroscopic data to further improve accuracy. For example, the processing system 106 may create an imaging loop based on the roadmap and fluoroscopic data to improve the navigation of the intraluminal device 102 through the vessels of the patient.

The processing system 106 receives echo data from the scanner assembly 110 by way of the PIM 104 and processes the data to reconstruct an image of the tissue structures in the medium surrounding the scanner assembly 110. Generally, the device 102 can be utilized within any suitable anatomy and/or body lumen of the patient. The processing system 106 outputs image data such that an image of the vessel or lumen 120, such as a cross-sectional IVUS image of the lumen 120, is displayed on the monitor 108. Lumen 120 may represent fluid filled or surrounded structures, both natural and man-made. Lumen 120 may be within a body of a patient. Lumen 120 may be a blood vessel, as an artery or a vein of a patient's vascular system, including cardiac vasculature, peripheral vasculature, neural vasculature, renal vasculature, and/or or any other suitable lumen inside the body. For example, the device 102 may be used to examine any number of anatomical locations and tissue types, including without limitation, organs including the liver, heart, kidneys, gall bladder, pancreas, lungs; ducts; intestines; nervous system structures including the brain, dural sac, spinal cord and peripheral nerves; the urinary tract; as well as valves within the blood, chambers or other parts of the heart, and/or other systems of the body. In addition to natural structures, the device 102 may be used to examine man-made structures such as, but without limitation, heart valves, stents, shunts, filters and other devices.

The controller or processing system 106 may include a processing circuit having one or more processors in communication with memory and/or other suitable tangible computer readable storage media. The controller or processing system 106 may be configured to carry out one or more aspects of the present disclosure. In some embodiments, the processing system 106 and the monitor 108 are separate components. In other embodiments, the processing system 106 and the monitor 108 are integrated in a single component. For example, the system 100 can include a touch screen device, including a housing having a touch screen display and a processor. The system 100 can include any suitable input device, such as a touch sensitive pad or touch screen display, keyboard/mouse, joystick, button, etc., for a user to select options shown on the monitor 108. The processing system 106, the monitor 108, the input device, and/or combinations thereof can be referenced as a controller of the system 100. The controller can be in communication with the device 102, the PIM 104, the processing system 106, the monitor 108, the input device, and/or other components of the system 100.

The processing system 106 is configured to calculate an expansion score, and optionally also a lumen score, for a stent based on received imaging data from the device 102, angiography system 130, external ultrasound system 132, and/or the CT system 134. These scores may be provided on a screen of the monitor 108 with other imaging data, such as one or more images of the lumen. The lesion score and the expansion score may be correlated to severity or effectiveness based on an established rating scale. These scores may help a user to accurately and quickly judge the lesion or stent and guide the user to an appropriate treatment. This may add confidence to the assessment of lesions and stents and save time in scoring procedures.

In some embodiments, the intraluminal device 102 includes some features similar to traditional solid-state IVUS catheters, such as the EagleEye^{®} catheter available from Volcano Corporation and those disclosed in U.S. Patent No. 7,846,101. For example, the intraluminal device 102 my include the scanner assembly 110 near a distal end of the intraluminal device 102 and a transmission line bundle 112 extending along the longitudinal body of the intraluminal device 102. The cable or transmission line bundle 112 can include a plurality of conductors, including one, two, three, four, five, six, seven, or more conductors.

The transmission line bundle 112 terminates in a PIM connector 114 at a proximal end of the intraluminal device 102. The PIM connector 114 electrically couples the transmission line bundle 112 to the PIM 104 and physically couples the intraluminal device 102 to the PIM 104. In an embodiment, the intraluminal device 102 further includes a guidewire exit port 116. Accordingly, in some instances the intraluminal device 102 is a rapid-exchange catheter. The guidewire exit port 116 allows a guidewire 118 to be inserted towards the distal end in order to direct the intraluminal device 102 through the lumen 120.

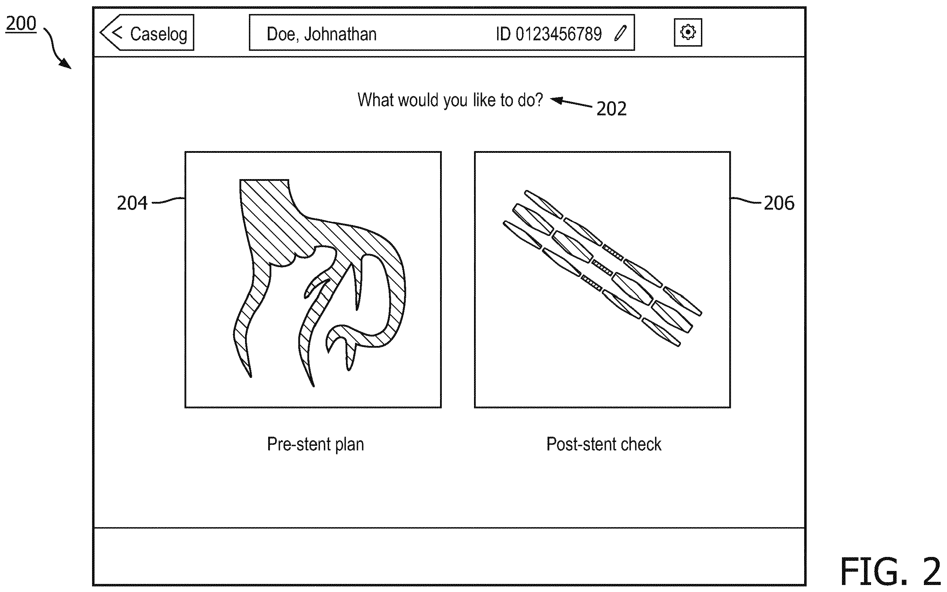

The monitor 108 may be a display device such as a computer monitor or other type of screen. The monitor 108 may be used to display selectable prompts, instructions, and visualizations of imaging data to a user. In some embodiments, the monitor 108 may be used to provide a procedure-specific workflow to a user to complete an intraluminal imaging procedure. This workflow may include performing a pre-stent plan to determine the state of a lumen and potential for a stent, as well as checking on a stent that has been positioned in a lumen. The workflow may be presented to a user as any of the displays or visualizations shown in Figs. 2-7.

Fig. 2 shows an exemplary display 200 showing a prompt 202 according to aspects of the present disclosure. In some embodiments, the display 200 is displayed on the monitor 108 as shown in Fig. 1. In other embodiments, the display 200 is displayed on a screen of another device, such as PIM 104. The display 200 may be generated by a controller of the intraluminal imaging system 100. In some embodiments, the display 200 is configured to display prompts and instructions as well as other data to an operator. The display 200 may be used to show a complete end-to-end workflow for an intraluminal procedure. This workflow may include a number of prompts and instructions that may guide an operator through a procedure. This may simplify the steps of a procedure and help to avoid operator errors.

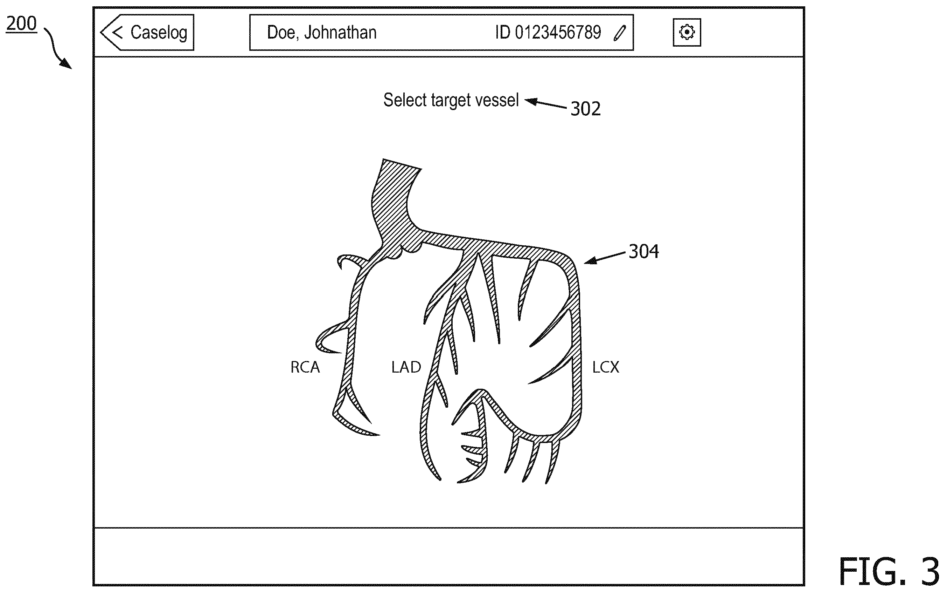

The prompts and instructions may be displayed on the display 200 as selectable options such that an operator may interact with the display 200 to choose options. The selections of the operator may change the display 200 such that information corresponding with the selected options is shown. In the example of Fig. 2, a selectable prompt 202 is displayed on display 200. The prompt includes two selectable options: option 204 corresponds to a pre-stent plan and option 206 corresponds to a post-stent check. The operator may select one of the options 204, 206 which may move the workflow forward, such that other screens are displayed (such as prompt 302 as shown in Fig. 3). The options 204, 206 may include visual representations of the type of procedure. For example, option 204 may include a depiction of vasculature within the heart and option 206 may include a depiction of a stent. In some embodiments, the selection of an option 204, 206 may involve a change in the visual depiction of the option 204, 206. For example, if the pre-stent plan option 204 is selected, the option 204 may appear as shaded or grey in future displays of the display 200. This may help to indicate that this option 204 has previously been selected by an operator. Other types of feedback may be used to indicate selections of options. For example, the selectable options 204, 206 may display blinking areas, highlighted areas, altered colors, shading, altered transparencies, and other visual indicators.

Option 204 may provide a workflow for a pre-stent plan that may include performing an intraluminal procedure (such as a pullback operation) and viewing results. Option 204 may be used to identify areas within a lumen 120 that may benefit from the placement of a stent. Option 206 may provide a workflow for a post-stent check that may include performing an intraluminal procedure (such as a pullback operation) and viewing results of an area within a lumen 120 where a stent has previously been placed. This option 206 may be used to observe the placement and effectiveness of the stent.

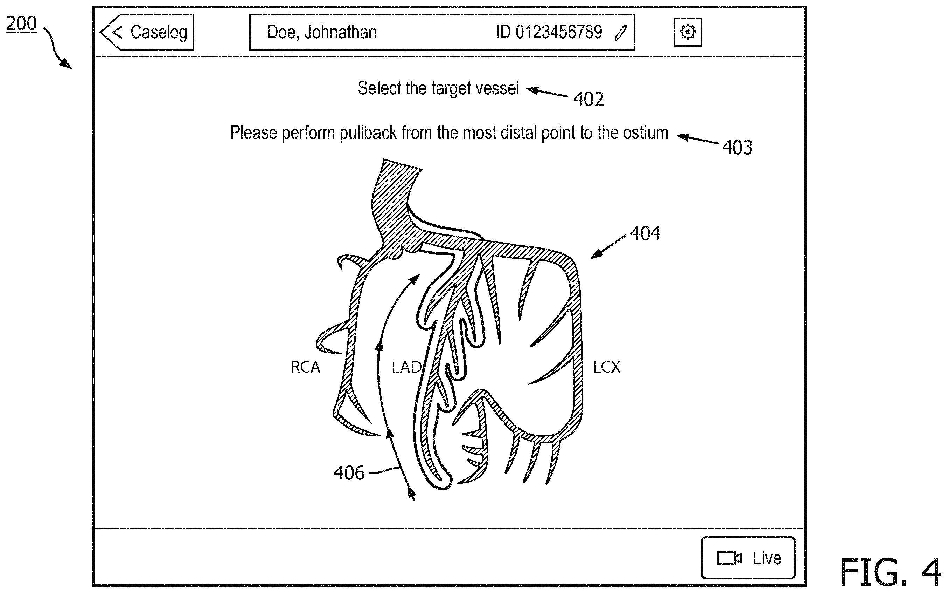

Fig. 3 shows an exemplary display 200 showing a prompt 302 according to aspects of the present disclosure. In some embodiments, the prompt 302 may be displayed after either of the options 204, 206 are selected. In other embodiments, the prompt 302 is displayed only after the pre-stent plan option 204 is selected. The prompt 302 may prompt the operator to select a target vessel. In the example of Fig. 3, selecting the target vessel includes selecting a region on a visualization 304 including arteries in the heart. The selectable regions may include the right coronary artery (RCA), left anterior descending (LAD), and left circumflex artery (LCX). The selectable regions may also include various regions of the arteries, as well as other vessels and lumens within other parts of the anatomy of a patient. The appearance of the visualization 304 may be altered when one of the regions is selected by the operator. For example, the selected artery may be outlined, highlighted, or colored with a different color. In some embodiments, the selected artery is outlined in blue, as shown in Fig. 4.

Fig. 4 shows an exemplary display 200 showing a prompt 402 according to aspects of the present disclosure. The prompt 402 may be displayed after the operator has made a selection on the prompt 302 shown in Fig. 3. In the example of Fig. 4, the LAD artery has been selected by an operator. The prompt 402 shows the outlined image of the LAD along with instructions 403 to perform a pullback procedure from the most distal point on the LAD to the ostium. These instructions 403 may refer to a pullback procedure or other movement of the device 102 within the selected vessel or lumen 120. The instructions 403 may instruct an operator to perform any type of movement of the device 102 within a selected target vessel. For example, the instructions 403 may instruct an operator to push the device 102 a given distance along the selected target vessel. A visualization 404 corresponding to the instructions 403 may also be displayed on the display 200. In the example of Fig. 4, the visualization 404 includes a blue line 406 with arrows showing the direction in which the pullback procedure should be performed. The visualization 404 may include visual effects such as changing colors or animation. For example, the arrows of the visualization 404 may move in the direction specified by the instructions 403. The instructions 403 and visualization 404 may vary depending on options that were previously selected. For example, if an operator selected the RCA as the target vessel, the visualization 404 of the RCA would be highlighted and a corresponding visualization would be displayed showing a procedure outlined by instructions 403.

In some embodiments, the instructions 403 of the display 200 may vary depending on which option 204, 206 was selected from the prompt 202 shown in Fig. 2. For example, if the post-stent check option 206 was selected, the instructions may read "please perform pullback from the distal point of the stent to the proximal point of the stent." Other instructions may also be included to guide the operator to perform an imaging procedure and acquire imaging data relevant to the selected target vessel and/or stent.

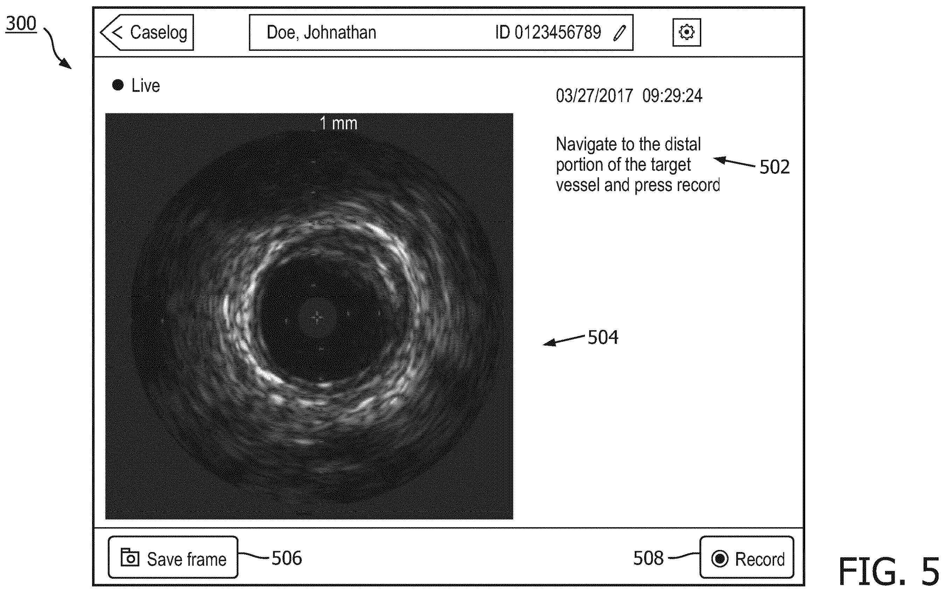

Fig. 5 shows an exemplary display 200 showing a prompt 502 according to aspects of the present disclosure. The prompt 502 may be displayed after the operator has made a selection on the prompt 402 shown in Fig. 4. In the example of Fig. 5, the LAD artery has been selected by an operator. The prompt 502 may be accompanied by a visualization 504. In some embodiments, the visualization 504 shows imaging data from the device 102 as the device 102 is moved through the selected target vessel. The imaging data may be used as a reference for the operator. In particular, imaging data shown in the visualization 504 may help the operator to know where to begin a procedure. In the example of Fig. 5, the imaging data may show when the device 102 is positioned at a distal end of the LAD artery so that a pullback operation may be performed. The imaging data may also show other reference data such as areas of interest along a lumen 120, branches of the lumen 120, problem areas within the lumen 120, and other features. In some embodiments, when the device 102 is placed at the location specified by the instructions (for example, at a distal portion of an artery), the operator may select the record button 508 to begin a recording of the procedure. The display may also include an option 506 to save specific frames of imaging data before or during a procedure.

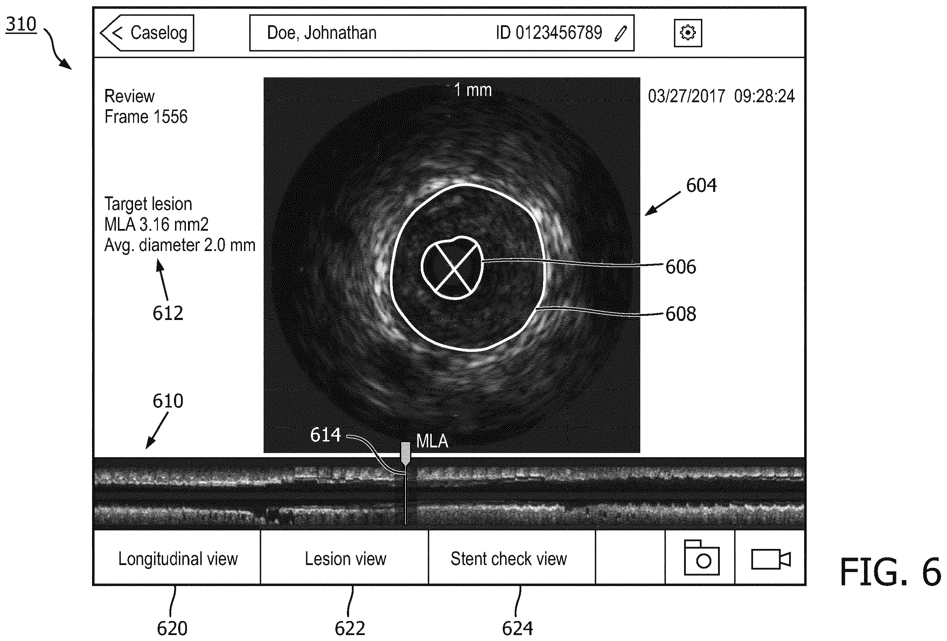

Fig. 6 shows an exemplary visualization 310 according to aspects of the present disclosure. The visualization 310 may be displayed on a monitor 108. The visualization 310 may present imaging data acquired by the device 102 during an intraluminal procedure. In some embodiments, the intraluminal procedure is outlined in the instructions shown in Figs. 3-5. In some embodiments, the visualization 310 includes imaging data corresponding to a lumen 120, such as the selected target vessel. The visualization 310 may include a first view 604 and a second view 610 of the lumen 120. In some embodiments, the first and second views 604, 610 may be oriented 90 degrees apart. In the example of Fig. 6, the first view 604 shows imaging data corresponding to a view straight down the lumen 120 (otherwise discussed as a "longitudinal view") and the second view 610 shows imaging data corresponding to a transverse view of the lumen 120. The views 604, 610 may include corresponding imaging data. The display of the first view 604 and second view 610 is not shown in existing systems, which generally include a single tomographic image. In other embodiments, other views may also be shown, including one or more transverse, cross-sectional, tomographic, 3D, or 4D images. For example, a 4D transverse image may be 3D transverse image with a time component shown.

In some embodiments, the visualization 310 may include a selected frame of imaging data received by the device 102. The operator may be able to select any frame from the imaging data received by the device 102. This may allow the operator to focus on specific areas of interest in the lumen 120.

In some embodiments, measurements are performed automatically on the imaging data with a controller of the intraluminal imaging system 100 as the imaging data is acquired by the device 102. Existing imaging systems typically require an operator to manually select a frame of interest and mark areas for measurement. This may be a time-consuming process, and may introduce user errors, especially in marking areas for measurement. These errors may cause operators to miss important features within the imaging data, such as lesions. The intraluminal imaging system 100 provides automated measurement of features in received imaging data without requiring user interaction. In some embodiments, the system 100 may automatically measure all applicable boundaries in the imaging data (including on a displayed image), including anatomical boundaries (such as lumen boundaries) and stents. Furthermore, the system 100 may automatically identify areas of interest based on the automatic measurements and display these areas of interest, correlated to a longitudinal view or angiographic image of the lumen. This automatic measurement, analysis, and display may provide an easy to understand overview of the lumen of the patient, as well as providing data for generating a lesion score.

In the example of Fig. 6, automatic measurements corresponding to a vessel boundary 608 and a minimum lumen area (MLA) 606 are displayed on the first view 604. The measurements may also include a vessel diameter, a center of the vessel, a vessel boundary 608 thickness, and other measurements performed automatically by the controller. These measurements may also be shown on other views. For example, a marker 614 is placed at the MLA in the second view 610 that corresponds with the MLA 606 in the first view 604. This may help an operator to visualize the diameter of vessel boundaries along the lumen 120. The measurements may be displayed in numerical format at box 612 on the visualization 310. Specific portions and views of the visualization 300 may be viewed by an operator by selecting the options 620, 622, and 624.

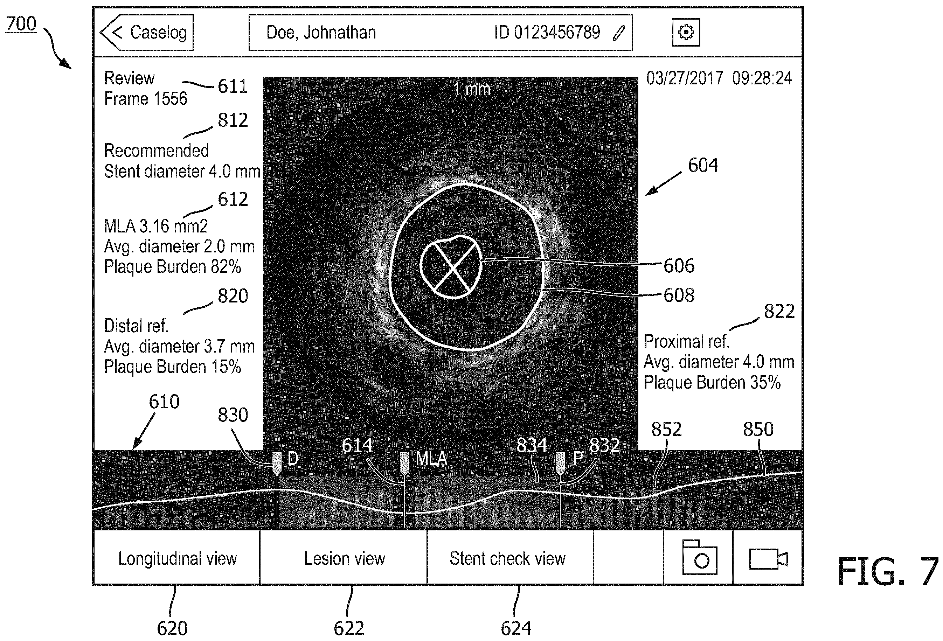

Fig. 7 shows an exemplary visualization 700 showing a lesion view according to aspects of the present disclosure. In some embodiments, visualization 700 corresponds to the pre-stent plan option 204 as shown in Fig. 2. In some embodiments, the visualization 700 may be used to recommend the placement and size of a stent to address a lesion. These recommendations may be made automatically by the system 100 based on the imaging data received by the device 102. In particular, the visualization 700 may be used to visualize a portion of a lumen 120 with a potential "landing zone" 834 for a stent. In some embodiments, the landing zone 834 is an area of interest within the lumen 120 that includes an MLA of a portion of the lumen 120, as marked by marker 614. The landing zone 834 may be shown in profile in view 610 to show the potential placement of the stent within the landing zone 834. A distal end marker 830 and a proximal end marker 832 of the landing zone 834 may define the distal and proximal extent of a potential stent. The distal end marker 830 and proximal end marker 832 may be accompanied with numerical data 820, 822 illustrating the average diameter and plaque burden of the lumen 120 at these locations. In some embodiments, the visualization may also a depiction of the plaque burden 852 along the lumen 120. In some embodiments, the depiction of the plaque burden 852 is automatically measured based on imaging data from the device 102. The visualization 700 may also include a depiction of lumen area 850. As illustrated in Fig. 7, the marker 614 for the MLA may be placed where the plaque burden is the greatest and the area of the lumen is the smallest. One or more of a plaque burden measurement, a lumen diameter measurement, a lumen area measurement, and other image- or physiology-based measurements may be used to generate a lesion score.

In some embodiments, the visualization 700 includes a recommended stent diameter as shown in text box 812. This diameter may be based on the diameter of the lumen 102 as measured by the system 100.

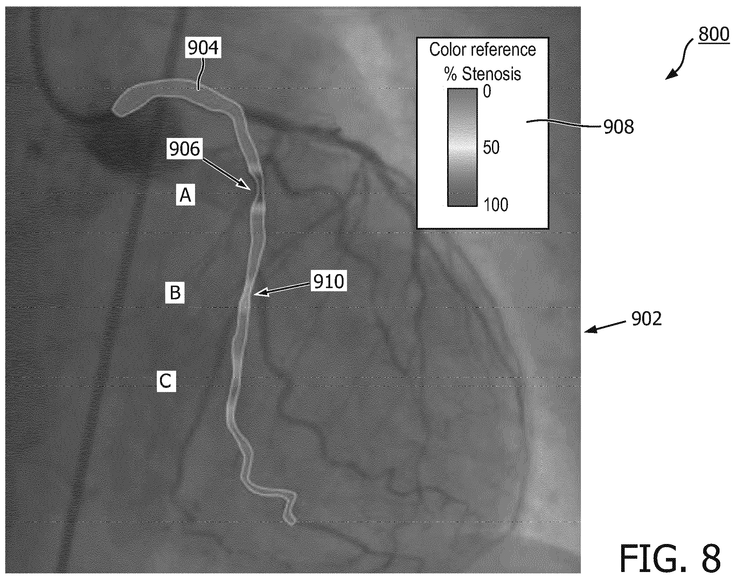

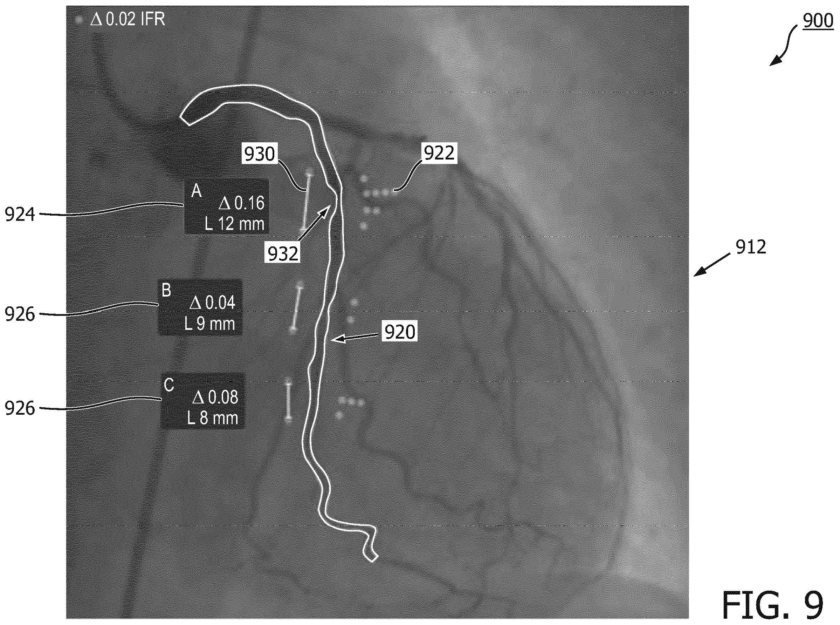

Fig. 8 shows an exemplary visualization 800 according to aspects of the present disclosure. The visualization 800 may be displayed on a monitor 108. The visualization 800 may include an image 902 of a lumen 910 and surrounding tissue. The image 902 may be a radiographic image, such as an angiographic image. In some embodiments, the lumen 910 includes a highlighted region 904. In some embodiments, the system 100 automatically measures and labels features of the lumen, including the lumen diameter and/or area. The highlighted region 904 may include one or more areas of interest 906 based on these measurements. In the example of Fig. 9, the highlighted region 904 includes three areas of interest 906, labeled with A, B, and C. In some embodiments, the highlighted region 904 may be colored according to percent stenosis. A color reference key 908 may be displayed in the angiographic image and may show the correlation between coloring in the highlighted region and percent stenosis. In the example of Fig. 8, the red coloring over the area of interest 906 indicates a lesion that is likely severe. Imaging data within the image 902 of Fig. 8 may be used to generate a lesion score. For example, the system 100 may analyze the color data within the highlighted region 904 and to determine that a lesion is present in the area of interest 906 that is likely severe.

Fig. 9 shows an exemplary visualization 900 according to aspects of the present disclosure. The visualization 900 may be displayed on a monitor 108. The visualization 800 may include an image 912 of a lumen 920 and surrounding tissue, which may be a radiographic image, such as an angiographic image. The lumen 910 may include measurements which may be automatically performed by the system 100. These measurements may include plaque burden, which may be displayed by symbols 922 along the length of the lumen 910. In some embodiments, the symbols 922 appear as dots representing the severity of the plaque burden in the lumen. Each dot may correspond to 10%, 15% or 20% plaque burden. In the example of Fig. 9, the plaque burden at the area of interest 932 is approximately 80%, indicating a fairly severe lesion. The lumen may also include one or more areas of interest 932 that may be automatically identified by the system 100 based on measurements of features in the lumen. In some embodiments, a text box 924, 924, 926 is displayed for each area of interest 932 in the image 912. These text boxes 924, 926, 928 may include a longitudinal measurement of the area of interest 932 (for example, 8, 9, or 12 mm), which may be shown with an arrow symbol 930 on the image 912. The text boxes 924, 926, 928 may also include a change in diameter across the lumen 910 (for example, 0.08, 0.04, and 0.16 mm) for each area of interest 932.

Fig. 10 shows an exemplary visualization 1000 with a lesion score 1002 according to aspects of the present disclosure. The visualization may include an image 1012 which may be an angiographic image including a lumen 920 and one or more intraluminal views 1010, 1020, 1030 of the lumen 920. In some embodiments, the intraluminal views 1010, 1020, 1030 are transverse views of the lumen 920 that show different positions along the lumen 920. For example, the lumen may include an area of interest 932 that is identified by the system 100 based on measured parameters of the lumen 920. A first intraluminal view 1010 may correlate to a distal reference position with respect to the area of interest 932, a second intraluminal view 1020 may correlate to a minimum lumen area (MLA) of the area of interest 932, and a third intraluminal view 1030 may correlate to a proximal reference position with respect to the area of interest 932. The intraluminal views 1010, 1020, 1030 may be generated by the system 100 after receiving imaging data from an intraluminal device 102 being moved through the lumen. In some embodiments, the intraluminal views 1010, 1020, 1030 are visually correlated to their positions along the angiographic view of the lumen 920, such as with dotted lines 1013 and matching colors as shown. Other visual correlations are possible, such as common symbols or indicators. The intraluminal views 1010, 1020, 1030 may provide a more complete view of the lumen 920 to give the user a more complete view of the lumen 920 and surrounding tissue.

In some embodiments, a lesion score 1002 is provided in the visualization 1000. The lesion score may be displayed in the image 1012, such as adjacent the area of interest 932. The lesion score 1002 may provide an indication of the severity of a lesion. Although the scoring of lesions and stent expansion is discussed, the scoring methods presented herein may be extended to other applications in vascular diagnosis, such as scoring the severity of an aneurism, thrombus, or other vascular malady as well as associated treatment scores. The lesion score 1002 may be calculated using one or more factors, including plaque burden, lumen area, lumen diameter, percent stenosis, eccentricity of the lumen, calcification within the lumen, position along the lumen (such as near a bifurcation), and or/pressure measurements within the lumen (such as FFR, IFR, PDPA). In some embodiments, the lesion score 1002 may also include factors relating to the angiographic image. For example, a ratio of the minimum width of an area of interest 932 in the lumen 920 relative to a reference length of the area of interest 932 (i.e., the distance between a distal reference point and a proximal reference point), a ratio of a blockage or lesion compared to other portions of the lumen 920, tortuosity in the lumen 920, and/or image quality issues such as areas of haziness. These factors may be weighted, such that factors that are more likely to be accurate are weighted higher than factors that are less likely to be accurate. In some embodiments, the factors related to invasive procedures (such as factors relating to IVUS data or other intravascular data) are weighted higher than factors relating to noninvasive procedures. The lesion score 1002 may be displayed with a highlighted area corresponding to the area of interest 932, as well as a maximum possible lesion score.

In some embodiments, the lesion score is represented by a number between 0 and 100, with 0 being least severe and 100 being most severe. In some embodiments, a high lesion score (such as 90 or 95) correlates to a severe lesion while a low score (such as 25 or lower) correlates to an insignificant lesion. In other embodiments, the lesion score may range from a value of 0.0 (least severe) to a value of 1.0 (most severe). Lesion scores may be correlated to visual cues such as color schemes (as shown by the highlighted region 904 in Fig. 8), such that red is severe and green is not severe. Other numbering systems, color schemes, and visual cues are also contemplated to represent the lesion score.

In some embodiments, a user may be able to select the lesion score 1002 and view the factors and/or weighting that went into its determination. The user may also be able to select the text box 924 and transverse views 1010, 1020, 1030 to access more information. In some embodiments, the transverse views 1010, 1020, 1030 may include highlighted boundaries of tissue layers, such as a vessel boundary 1016 and a lumen boundary 1018. The image 1012, transverse views 1010, 1020, 1030, and lesion score 1002 may help a user to more clearly understand the layout of a lumen 920 and surrounding tissue as well as more clearly understand the severity of lesions within the lumen 920.

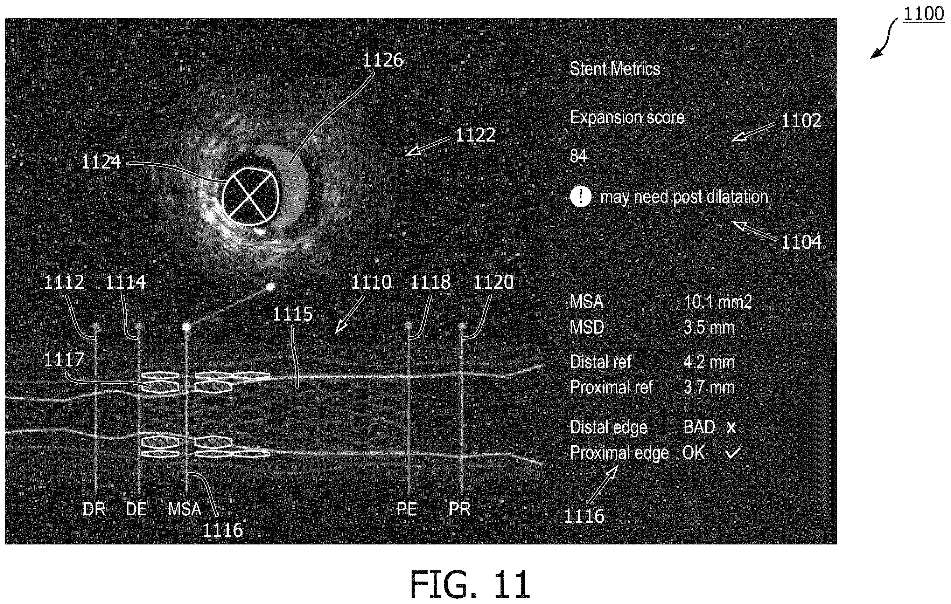

Fig. 11 shows an exemplary visualization 1100 showing a stent within a lumen according to aspects of the present disclosure. In some embodiments, the visualization 1100 is shown after the operator has selected the stent check option 204 of Fig. 2 and has been guided through the subsequent workflow steps. The visualization 1100 may display imaging data gathered from the device 102 during motion within a lumen 120 (such as a pullback procedure) where a stent has been placed, as well as imaging data of surrounding areas of the lumen. The visualization 1100 may include a longitudinal view 1110 of the vessel and stent as well as a transverse view 1122 of the vessel and stent. The system 100 may automatically measure features in the imaging data, such as lumen boundaries, vessel boundaries, and stent edges and boundaries. For example, the shape and size of a boundary 1124 of the stent may be measured and displayed in the transverse view 1122. The shape and size of the stent may be measured and displayed as a depiction of the stent 1115 in the longitudinal view 1110. The visualization 1100 may include measurements of the diameter, area, and length of the stent. These measurements may be displayed in the stent metrics box 1106. In the example of Fig. 11, the minimum stent area (MSA) has been measured as 10.1 mm² and the minimum stent diameter (MSD) has been measured as 3.5 mm.

The visualization 1100 may include indicators 1114, 1118 marking the distal and proximal edges of the stent based on the received imaging data, as well as indicators 1112, 1120 marking distal and proximal reference points. These reference points may be automatically determined by the system 100 based on percent stenosis measured within the lumen, such as 30 percent stenosis or more. In other embodiments, the distal and proximal references are determined by the user. The indicators 1112, 1120 may be moved along the lumen by the user to view different sets of imaging data. The visualization 1100 may also include an indicator 1116 marking the MSA of the stent. The transverse view 1122 may correspond to this indicator as shown in Fig. 11. The user may also be able to select any of the other indicators 1112, 1114, 1118, 1120 to access a transverse view with data corresponding to the position of the selected indicator 1112, 1114, 1118, 1120 in the vessel.

In some embodiments, an expansion score 1102 may be provided in the visualization 1100. The expansion score may be included in the stent metrics box 1106. The expansion score 1102 may be calculated using one or more factors. In some embodiments, the expansion score is based on how closely struts 1117 of the stent 1115 align with the lumen wall. In particular, the more struts 1117 that are significantly offset from the lumen wall, the lower the expansion score. The stent struts 1117 may be depicted in the visualization, including areas of red highlighting where the stent struts 1117 are not aligned with the lumen wall. Misalignment between the stent 1115 and lumen may also be shown in the transverse view 1122, such as by highlighted area 1126. The expansion score may also be based on other factors including alignment other features of the stent with the lumen walls, expansion at the edges of the stent, measured stent area, diameter and length, areas of misalignment or malapposition, positioning of the stent along the lumen, and or/pressure measurements within the lumen (such as FFR, IFR, PDPA). These factors may be weighted, such that factors that are more likely to be accurate are weighted higher than factors that are less likely to be accurate. In some embodiments, the factors related to invasive procedures (such as factors relating to IVUS data or other intravascular data) are weighted higher than factors relating to noninvasive procedures.

In some embodiments, the expansion score is represented by a number between 0 and 100, with 0 being completely unexpanded and 100 being completely expanded. In other embodiments, the expansion score may range from a value of 0.0 (not expanded) to a value of 1.0 (completely expanded). Similar to a lesion score, an expansion score may be correlated to visual cues such as color schemes such that red represents misalignment of the stent (as shown in Fig. 11) and black represents full alignment of the stent. This color scheme may vary across the image of the stent so that a user may see which areas of stent are not aligned with the lumen walls. Other numbering systems, color schemes, and visual cues are also contemplated to represent the expansion score.

The expansion score 1102 may be displayed with a highlighted area corresponding an area of malapposition 1126 as shown in Fig. 11. Areas of malapposition may be shown in both the transverse view 1122 and the longitudinal view 1110 to give the user a clear view of problems with the stent. A recommendation 1104 may be displayed in the visualization 1100 based on the expansion score 1102. In the example of Fig. 11, the recommendation is "may need post dilation" based on an expansion score of 84. In some embodiments, the recommendation 1104 may include post dilation for expansion scores under 90 percent, 85 percent, 80 percent, or 75 percent. Other recommendations 1104 may also be provided, such as reimaging certain areas of the lumen. The visualization 1100 may include a determination of stent edge placement. In the example of Fig. 11, the distal edge is determined to be "bad" while the proximal edge is determined to be "ok." The stent edge determination may be based on expansion of the stent walls adjacent the stent edge. For example, the stent edge may be determined to be "bad" if the expansion is below 90, 85, 80, or 75 percent. The visualization 1100 along with the expansion score 1102 may help a user to more clearly understand the placement and expansion of a stent within the lumen.

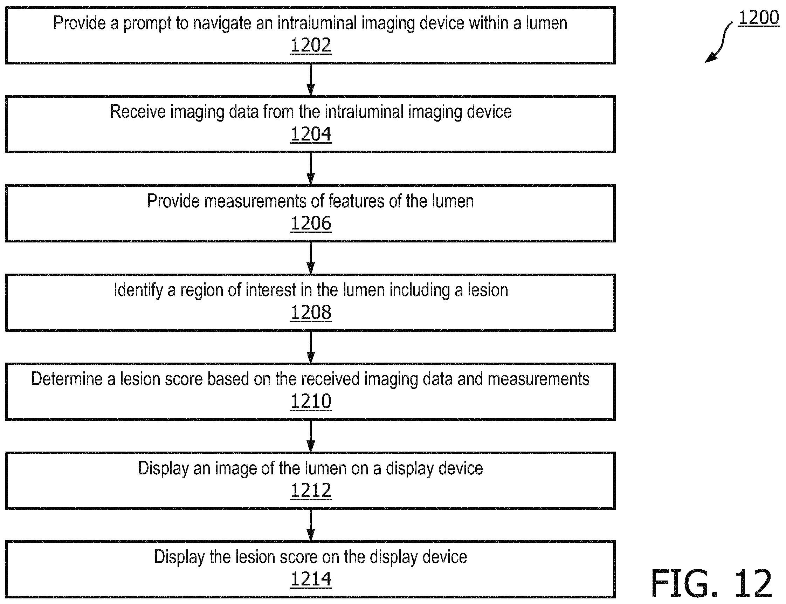

Fig. 12 is a flow diagram of a method 1200 of proving a guided workflow for an intraluminal imaging procedure to a user. In some embodiments, the steps of the method 1200 may be carried out by the intraluminal imaging system 100 and associated components as shown in Fig. 1 and any of the displays as shown in Figs. 5-11. It is understood that the steps of method 1200 may be performed in a different order than shown in Fig. 12, additional steps can be provided before, during, and after the steps, and/or some of the steps described can be replaced or eliminated in other embodiments.

At step 1202, the method 1200 may include providing a prompt to navigate an intraluminal imaging device within a lumen. The intraluminal imaging device may be the intraluminal imaging device 102 as shown in Fig. 1. The prompt may include navigating the intraluminal imaging device to a starting point in the lumen, as well as activating sensors in the intraluminal device. This prompt may be presented with text as well as images showing where the user should place the intraluminal device.

At step 1204, the method 1200 may include receiving imaging data from the intraluminal device. This imaging data may help a user to accurately navigate the intraluminal device according to the prompt of step 1202. For example, if the prompt of step 1202 directs the user to navigate the intraluminal device from a distal end of the lumen to a proximal end of the lumen, the imaging data may show imaging data from the intraluminal device as it is moved through the lumen. In some embodiments, the imaging data may include IVUS data showing the layers of tissue on the interior of the vessel. In other embodiments, the imaging data includes data from another modality such as angiographic image data. This data may be used to compile an angiographic image of the lumen. Thus, the imaging data may help the user to accurately perform the operation outlined in the prompt.

At step 1206, the method 1200 may include providing measurements of features within the lumen. This step may include automatically or manually measuring features within the two or more views with the system. In some embodiments, the system may automatically identify features based on variations in imaging data and automatically measure the dimensions of these features. In other embodiments, a user may identify features within the views and manually measure the dimensions of these features. The features may include anatomical features such as tissue boundaries, lesions, bifurcations, *etc.,* as well as manmade features such as stents. In some embodiments, the measurements include a diameter and area of the lumen along its length, as well as percent stenosis of the lumen along its length. The automatic measurements may be carried out on intraluminal images of the lumen as well as radiographic images, such as angiographic images of the lumen.

At step 1208, the method 1200 may include identifying a region of interest in the lumen including a lesion. The region of interest may be automatically identified based on the measurements of step 1206. For example, a region of interest may be identified around an MLA. The identified region of interest may be displayed on a display device such as monitor 108 shown in Fig. 1. The region of interest may be correlated to the longitudinal or angiographic image of the lumen.

At step 1210, the method 1200 may include determining a lesion score based on the received imaging data and manual or automatic measurements. The lesion score may be based on factors including plaque burden, lumen area, lumen diameter, percent stenosis, eccentricity of the lumen, calcification within the lumen, position along the lumen (such as near a bifurcation), and or/pressure measurements within the lumen (such as FFR, IFR, PDPA). In some embodiments, the lesion score is based on received measurements from a pressure-sensing guide wire and an IVUS imaging device together. The lesion score may be represented by a number between 0 and 100, with 0 being least severe and 100 being most severe.

At step 1212, the method 1200 may include displaying an image of the lumen on a display device. In some embodiments, two or more views of the lumen are shown on a same screen of the display device. For example, a transverse intraluminal view of the lumen may be shown with an angiographic view of the lumen. A transverse intraluminal view of the lumen may also be shown with a longitudinal intraluminal view of the lumen. The views of the lumen may be visually correlated, such that a user can easily understand which portions of the lumen are being displayed. Any of the views of the lumen as shown in Figs. 5-11 may be displayed at step 1212.

At step 1214, the method 1200 may include displaying the lesion score on the display device. In some embodiments, the lesion score may be displayed on same screen as one or more images of the lumen as discussed in step 1212. In some embodiments, the lesion score is displayed with an angiographic image of the lumen and one or more transverse intraluminal views of the lumen. The lesion score may be displayed with a recommendation, such as a recommended imaging procedure or treatment based on the lesion score.

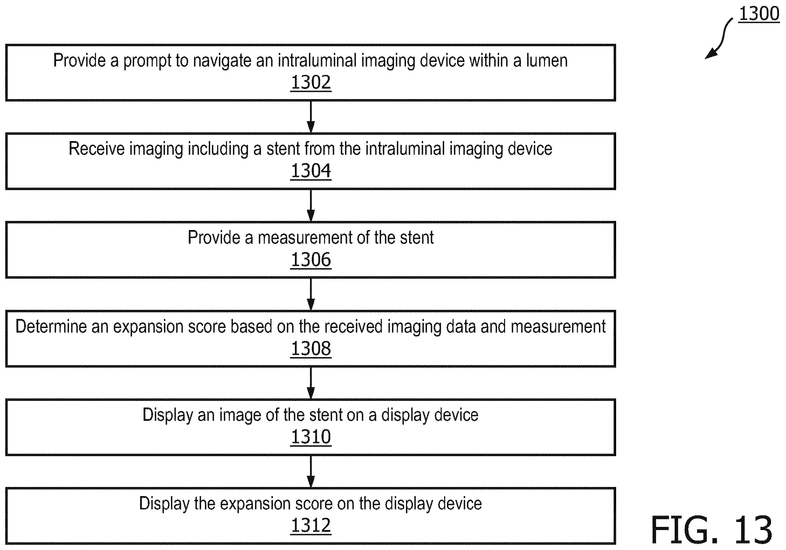

Fig. 13 is a flow diagram of a method 1300 of proving a guided workflow for an intraluminal imaging procedure to a user. In some embodiments, the steps of the method 1300 may be carried out by the intraluminal imaging system 100 and associated components as shown in Fig. 1 and any of the displays as shown in Figs. 5-11. It is understood that the steps of method 1300 may be performed in a different order than shown in Fig. 13, additional steps can be provided before, during, and after the steps, and/or some of the steps described can be replaced or eliminated in other embodiments.

At step 1302, the method 1300 may include providing a prompt to navigate an intraluminal imaging device within a lumen. The intraluminal imaging device may be the intraluminal imaging device 102 as shown in Fig. 1. The prompt may include navigating the intraluminal imaging device to a starting point in the lumen, as well as activating sensors in the intraluminal device. This prompt may be presented with text as well as images showing where the user should place the intraluminal device.

At step 1304, the method 1300 may include receiving imaging data from the intraluminal device including a stent. This data may be used to compile an angiographic image of the lumen and stent. Thus, the imaging data may help the user to accurately perform the operation outlined in the prompt.

At step 1306, the method 1300 may include providing measurement of the stent. This step may include automatically or manually measuring the stent within the two or more views with the system. In some embodiments, the system may automatically identify the stent based on variations in imaging data and automatically measure the dimensions of the stent. In other embodiments, a user may identify the stent within the views and manually measure the dimensions of the stent. These measurements may include a length of the stent, the position of the stent within the lumen, the alignment of the stent walls and the lumen walls, and/or an area and diameter of the stent along its length. The automatic measurements may be carried out on intraluminal images of the lumen and stent as well as radiographic images, such as angiographic images of the lumen and stent.

At step 1308, the method 1300 may include determining an expansion score based on the received imaging data and manual or automatic measurements. The expansion score may be based on one or more factors including alignment of the stent walls with the lumen walls, expansion at the edges of the stent, measured stent area, diameter and length, areas of misalignment or malapposition, positioning of the stent along the lumen, and or/pressure measurements within the lumen (such as FFR, IFR, PDPA). In some embodiments, the expansion score is represented by a number between 0 and 100, with 0 being completely unexpanded severe and 100 being completely expanded.

At step 1310, the method 1300 may include displaying an image of the stent on a display device. In some embodiments, the stent is displayed within a lumen. Two or more views of the lumen and stent may be shown on a same screen of the display device. For example, a transverse intraluminal view of the stent and lumen may be shown with an angiographic view of the stent and lumen. A transverse intraluminal view of the stent and lumen may also be shown with a longitudinal intraluminal view of the stent and lumen. The views of the stent and lumen may be visually correlated, such that a user can easily understand which portions of the lumen are being displayed. Any of the views of the lumen as shown in Figs. 5-11 may be displayed at step 1310.

At step 1312, the method 1300 may include displaying the expansion score on the display device. In some embodiments, the expansion score may be displayed on same screen as one or more images of the stent and lumen as discussed in step 1310. In some embodiments, the expansion score is displayed with an angiographic image of the stent and lumen and one or more transverse intraluminal views of the stent and lumen. The expansion score may be displayed with a recommendation, such as a recommended imaging procedure or treatment based on the expansion score.

Persons skilled in the art will recognize that the apparatus, systems, and methods described above can be modified in various ways. Accordingly, persons of ordinary skill in the art will appreciate that the embodiments encompassed by the present disclosure are not limited to the particular exemplary embodiments described above.

## Claims

1. An intraluminal imaging system (100), comprising:
a controller in communication with an intraluminal imaging device (102) positionable within a body lumen of a patient, the controller configured to:
receive, from the intraluminal imaging device, imaging data associated with a stent positioned within the body lumen;
provide a measurement of the stent based on the received imaging data;
determine an expansion score for the stent based on the imaging data and the measurement of the stent; and
a display device in communication with the controller, the display device configured to display, on a single screen, the expansion score, the image of the stent in a longitudinal image of the body lumen, a transverse view of the body lumen based on the received imaging data and a recommendation of an imaging procedure or treatment based on the expansion score;
wherein the longitudinal image of the body lumen comprises an indicator (1116) marking a minimum stent area and wherein the transverse view (1122) comprises a shape and a size of a boundary of the stent (1124) at the position that corresponds to the indicator.

2. The intraluminal imaging system of claim 1, wherein the longitudinal image includes a first indicator indicating a distal reference point (1112) of the stent, a second indicator indicating a proximal reference point (1120) of the stent, a third indicator (1114) and a fourth indicator (1118) marking the distal and proximal edges of the stent.

3. The intraluminal imaging system of claim 1, wherein the display further includes a highlighted region (1126) in the transverse view, showing a misalignment between the stent and the body lumen.

4. The intraluminal imaging system of claim 1, wherein the measurement is a lumen area of the stent.

## Patentansprüche

1. Intraluminales Bildgebungssystem (100), umfassend:
eine Steuereinheit, die mit einer intraluminalen Bildgebungsvorrichtung (102) kommuniziert, die innerhalb eines Körperlumen eines Patienten positionierbar ist, wobei die Steuereinheit dazu konfiguriert ist:
von der intraluminalen Bildgebungsvorrichtung Bildgebungsdaten zu empfangen, die mit einem innerhalb des Körperlumen positionierten Stent verbunden sind;
basierend auf den empfangenen Bildgebungsdaten eine Messung des Stents bereitzustellen;
basierend auf den Bildgebungsdaten und der Messung des Stents einen Expansionswert für den Stent zu bestimmen; und
eine Anzeigevorrichtung, die mit der Steuereinheit kommuniziert, wobei die Anzeigevorrichtung dazu konfiguriert ist, auf einem einzelnen Bildschirm den Expansionswert, das Bild des Stents in einem Längsbild des Körperlumens, eine Queransicht des Körperlumens basierend auf den empfangenen Bildgebungsdaten und eine Empfehlung für eine Bildgebungsprozedur oder eine Behandlung basierend auf dem Expansionswert anzuzeigen;
wobei das Längsbild des Körperlumens einen Indikator (1116) umfasst, der eine minimale Stentfläche markiert, und wobei die Queransicht (1122) eine Form und eine Größe einer Begrenzung des Stents (1124) an der Position umfasst, die dem Indikator entspricht.

2. Intraluminales Bildgebungssystem nach Anspruch 1, wobei das Längsbild einen ersten Indikator, der einen distalen Referenzpunkt (1112) des Stents angibt, einen zweiten Indikator, der einen proximalen Referenzpunkt (1120) des Stents angibt, einen dritten Indikator (1114) und einen vierten Indikator (1118), die den distalen und den proximalen Rand des Stents markieren, einschließt.

3. Intraluminales Bildgebungssystem nach Anspruch 1, wobei die Anzeige weiter einen hervorgehobenen Bereich (1126) in der Queransicht einschließt, der eine Fehlausrichtung zwischen dem Stent und dem Körperlumen zeigt.

4. Intraluminales Bildgebungssystem nach Anspruch 1, wobei die Messung eine Lumenfläche des Stents ist.

## Revendications

1. Système d'imagerie intraluminale (100), comprenant :
un contrôleur en communication avec un dispositif d'imagerie intraluminale (102) positionnable dans une lumière corporelle d'un patient, le contrôleur étant configuré pour :
recevoir, en provenance du dispositif d'imagerie intraluminale, des données d'imagerie associées à un stent positionné dans la lumière corporelle ;
fournir une mesure du stent basée sur les données d'imagerie reçues ;
déterminer un score d'expansion pour le stent en fonction des données d'imagerie et des mesures du stent ; et
un dispositif d'affichage en communication avec le contrôleur, le dispositif d'affichage étant configuré pour afficher, sur un seul écran, le score d'expansion, l'image du stent dans une image longitudinale de la lumière corporelle, une vue transversale de la lumière corporelle basée sur les données d'imagerie reçues et une recommandation d'une procédure d'imagerie ou d'un traitement reposant sur le score d'expansion ;
dans lequel l'image longitudinale de la lumière corporelle comprend un indicateur (1116) marquant une surface minimale de stent et dans lequel la vue transversale (1122) comprend une forme et une taille d'une limite du stent (1124) à la position qui correspond à l'indicateur.

2. Système d'imagerie intraluminale selon la revendication 1, dans lequel l'image longitudinale inclut un premier indicateur indiquant un point de référence distal (1112) du stent, un deuxième indicateur indiquant un point de référence proximal (1120) du stent, un troisième indicateur (1114) et un quatrième indicateur (1118) marquant les bords distal et proximal du stent.

3. Système d'imagerie intraluminale selon la revendication 1, dans lequel l'affichage inclut en outre une région mise en évidence (1126) dans la vue transversale, montrant un désalignement entre le stent et la lumière corporelle.

4. Système d'imagerie intraluminale selon la revendication 1, dans lequel la mesure est une surface luminale du stent.
